(19) Europäisches Patentamt
European Patent Office
Office européen des brevets

(11) EP 4 588 360 A1

(12) **EUROPEAN PATENT APPLICATION**
published in accordance with Art. 153(4) EPC

(43) Date of publication:
23.07.2025 Bulletin 2025/30

(21) Application number: 23865462.8

(22) Date of filing: 11.09.2023

(51) International Patent Classification (IPC):
A23L 5/00 (2016.01)     A23C 11/10 (2025.01)
A23L 2/00 (2006.01)     A23L 2/38 (2021.01)
A23L 2/52 (2006.01)     A23L 7/10 (2016.01)
A23L 7/104 (2016.01)    A23L 11/60 (2025.01)
A23L 11/65 (2025.01)    C12N 9/26 (2006.01)
C12N 9/42 (2006.01)

(52) Cooperative Patent Classification (CPC):
A23C 11/10; A23L 2/00; A23L 2/38; A23L 2/52;
A23L 5/00; A23L 7/10; A23L 7/104; A23L 11/60;
A23L 11/65; C12N 9/2408; C12N 9/2434

(86) International application number:
PCT/JP2023/033012

(87) International publication number:
WO 2024/058109 (21.03.2024 Gazette 2024/12)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
KH MA MD TN

(30) Priority: 16.09.2022 JP 2022147735

(71) Applicant: Amano Enzyme Inc.
Nagoya-shi
Aichi 460-8630 (JP)

(72) Inventors:
• TABUSE, Shiho
  Kakamigahara-shi, Gifu 509-0109 (JP)
• TAKAYAMA, Hiroyuki
  Kakamigahara-shi, Gifu 509-0109 (JP)

(74) Representative: WP Thompson
138 Fetter Lane
London EC4A 1BT (GB)

(54) **ENZYME AGENT FOR REDUCING FLOURY TASTELESSNESS OF PLANT-DERIVED BEVERAGE OR FOOD OR PLANT-DERIVED BEVERAGE OR FOOD MATERIAL**

(57)     [Problems] To provide plant-base milk with reduced powdery feel and altered taste.

[Solution] The present technique provides an enzyme agent for reducing the powdery feel of a plant-base beverage or food or a plant-base beverage or food material, which contains a cell wall polysaccharide degrading enzyme, and a plant-base beverage or food or a plant-base beverage or food material using the enzyme agent for reducing powdery feel. The present technique also provides a method for producing a plant-base beverage or food or a plant-base beverage or food material, and a method for reducing the powdery feel of a plant-base beverage or food or a plant-base beverage or food material, which include a step of allowing a cell wall polysaccharide degrading enzyme to act on a plant-base raw material.

EP 4 588 360 A1

## Description

Technical Field

The present technique relates to an enzyme agent for reducing the powdery feel of a plant-base beverage or food or a plant-base beverage or food material. More specifically, the present technique relates to an enzyme agent for reducing the powdery feel of a plant-base beverage or food or a plant-base beverage or food material, a plant-base beverage or food or a plant-base beverage or food material using the enzyme agent for reducing powdery feel, and, a method for producing a plant-base beverage or food or a plant-base beverage or food material, and a method for reducing the powdery feel of a plant-base beverage or food or a plant-base beverage or food material.

Background Art

In recent years, plant-base beverages or foods have been attracting attention due to the growing health consciousness. Unlike animal milk, which is a secretion liquid, plant-base milk is prepared by dispersing crushed plant tissue in water, and therefore, as a basic characteristic of a substitute for animal milk, it is required to have smooth mouthfeel. In addition, in producing plant-base milk, pleasant flavor derived from raw materials may be lost, and the overall flavor may be adversely affected. For this reason, improvements in food texture and taste of plant-base milk are required.

Various technologies have been studied for the purpose of imparting characteristics closer to animal milk to plant-base milk. For example, Patent Literature 1 describes a technique for producing a cereal liquefied product that achieves both low viscosity and reduced free sugar content by allowing 4-$\alpha$-glucanotransferase, cellulase, and peptidase to act on oats. Patent Literature 2 describes a technique for producing an oat drink with moderate sweetness by degrading starch in oat materials with one or more amylases. Patent Literature 3 describes that the nutritional components are changed by allowing phytase to act on oat bran, and that the mouthfeel of a food composition is improved by a micronization step. Patent Literature 4 describes that a rice dispersion is treated with protease, cellulase, pullulanase, and $\alpha$-amylase to produce a rice liquefied product that does not exhibit sweetness and is rich in oligosaccharides, and that a homogenization treatment is further performed to make the rice liquefied product have smooth food texture.

Citation List

Patent Literature

Patent Literature 1: JP-A No. 2021-40553
Patent Literature 2: JP-A No. 2018-075029
Patent Literature 3: JP-A No. 2015-84687
Patent Literature 4: JP-A No. 2004-121135

Summary of the Invention

Technical Problem

Because plant-base milk has a different composition from animal-derived milk, there is still room for improvement before it can achieve satisfactory characteristics. For example, in order to make the food texture smooth, special equipment is required for micronization and homogenization, so a simpler method is desired. In addition, with regard to taste, a method of changing the taste without adding unnecessary raw materials is desired due to diversifying needs and health consciousness.

Therefore, the main objective of this technique is to provide plant-base milk with reduced powdery feel and changed taste.

Solution to Problem

As a result of intensive research, the inventors of the present application found that the powdery feel can be reduced by allowing a cell wall polysaccharide degrading enzyme to act on plant-base milk. Based on this finding, the present technique was completed through further research.

That is, the present technique first provides an enzyme agent for reducing the powdery feel of a plant-base beverage or food or a plant-base beverage or food material, the enzyme including a cell wall polysaccharide degrading enzyme.

The present technique also provides a plant-base beverage or food, or a plant-base beverage or food material, which uses the enzyme agent for reducing powdery feel according to the present technique.

The plant-base beverage or food according to the present technique may be oat milk or rice milk.

Next, the present technique provides a method for producing a plant-base beverage or food or a plant-base beverage or food material, which includes a step of allowing a cell wall polysaccharide degrading enzyme to act on a plant-base raw material.

The present technique further provides a method for reducing the powdery feel of a plant-base beverage or food or a plant-base beverage or food material, the method including a step of allowing a cell wall polysaccharide degrading enzyme to act on a plant-base raw material.

Advantageous Effects of Invention

According to the present technique, a plant-base beverage or food or a plant-base beverage or food material that has reduced powdery feel and altered taste is provided.

Description of Embodiments

A preferred embodiment for carrying out the present technique will be described below. Note that the embodiment described below shows an example of a typical embodiment of the present technique, and the scope of the present technique is not to be interpreted narrowly by this.

1. Enzyme agent for reducing powdery feel

The enzyme agent for reducing powdery feel according to the present technique contains cell wall polysaccharide degrading enzyme as active ingredients. Carbohydrates are made up of sugars including polysaccharides such as starch, oligosaccharides, monosaccharides, disaccharides, etc., and dietary fiber including cell wall polysaccharides. Cell wall polysaccharides include cellulose, hemicellulose, pectin, etc., and the enzyme that degrades these cell wall polysaccharides is the cell wall polysaccharide degrading enzyme and the like.

The cell wall polysaccharide degrading enzyme that can be used in the present technique is not particularly limited as long as it has the ability to degrade cell wall polysaccharides, and one or more types of cell wall polysaccharide degrading enzymes that can be used in the production of beverages or foods can be used in any combination as long as the effect of the present technique is not impaired. Examples of cell wall polysaccharide degrading enzymes include cellulase, hemicellulase, and pectinase. Among these, it is particularly preferable to use cellulase in the enzyme agent for reducing powdery feel according to the present technique. The enzymes that can be used in the enzyme agent for reducing powdery feel according to the present technique will be described in detail below.

(1) Cellulase

The cellulase that can be used in the present technique is an enzyme that hydrolyzes the glycosidic bonds of β-1,4-glucan and breaks down cellulose, which is one of the cell wall polysaccharides. The cellulase that can be used in the present technique may be an enzyme that also has other functions as long as it has cellulase activity. In the present technique, the powdery feel of the produced plant-base beverage or food can be reduced by allowing cellulase to act on a plant-base raw material.

The origin of cellulase that can be used in the present technique is not particularly limited, and examples thereof include microorganisms belonging to the genus Aspergillus, such as Aspergillus niger, Aspergillus oryzae, Aspergillus sojae, Aspergillus saitoi, Aspergillus awamori, and Aspergillus flavus; the genus Trichoderma, such as Trichoderma reesei and Trichoderma viride; and the genus Acremonium, such as Acremonium cellulolyticus. One type of these cellulases may be used alone, or several types may be used in combination. Among these, from the viewpoint of reducing the powdery feel of the plant-base raw material, it is more preferable that the cellulase is derived from a microorganism of the genus Trichoderma, in particular from Trichoderma viride.

Here, "cellulase derived from Trichoderma viride" means cellulase generated by a microorganism (whether a wild type or a mutant strain) classified as Trichoderma viride, or cellulase obtained by a genetic engineering approach using a cellulase gene. Therefore, a recombinant generated by a host microorganism into which a cellulase gene obtained from Trichoderma viride (or a gene obtained by modifying said gene) has been introduced also falls under "cellulase derived from Trichoderma viride".

The type of cellulase used is not particularly limited and may be, for example, endoglucanase, exoglucanase, or a mixture thereof. The cellulase used in the present invention is preferably an enzyme agent containing both endoglucanase and exoglucanase.

The cellulase used in the present technique can be prepared from the culture solution of the microorganism from which the above-mentioned cellulase is derived. Specific preparation methods include a method of recovering cellulase from the culture solution or cells of the above-mentioned microorganism. For example, when a cellulase-secreting microorganism is used, the cells can be recovered from the culture solution in advance by filtration, centrifugation, or the like as necessary, and then the enzyme can be separated and/or purified. When a cellulase-non-secreting microorganism is used, the cells can be recovered from the culture solution in advance as necessary, and then the cells can be crushed by pressure treatment, ultrasonic treatment, or the like to expose the enzyme, and then the enzyme can be separated and/or purified. As a method for separating and/or purifying the enzyme, a known protein separation and/or purification method can be used without any particular limitation, and examples of the method include centrifugation, UF concentration, salting out, and various chromatography methods using ion exchange resins, etc. The separated and/or purified enzyme can be powdered by a drying method such as freeze-drying or vacuum drying, and can also be powdered using an appropriate excipient and/or drying aid in the drying method. In addition, the separated and/or purified enzyme can be liquefied by adding appropriate additives and sterilizing by filtration.

In the present technique, commercially available cellulases can also be used, and preferred examples of commercially available cellulases include cellulase derived from Trichoderma viride manufactured by Amano Enzyme Inc.

The content of cellulase in the powdery feel reducing enzyme agent according to the present technique can be freely set as long as it does not impair the effect of the present technique. The content of cellulase can be set to, for example, 0.01 U or more per 1 g of the plant-base raw material used in the plant-base beverage or food or plant-base beverage or food material to be produced, and from the viewpoint of further enhancing the powdery feel reducing effect, it can be set to preferably 0.05 U or more, more preferably 0.1 U or more, even more preferably 0.3 U or more, and even more preferably 0.5 U or more.

The upper limit of the cellulase content is not particularly limited as long as it does not impair the effects of the present technique, but can be set to, for example, 500 U or less, 200 U or less, 100 U or less, 50 U or less, 30 U or less, 20 U or less, or 10 U or less per 1 g of plant-base raw material used in the plant-base beverage or food or plant-base beverage or food material to be produced.

In the present technique, the cellulase activity is a value defined by the following method.

[Definition of cellulase activity]

Regarding the activity of cellulase, the amount of enzyme that brings about an increase in reducing power equivalent to 1 $\mu$mole of glucose per minute is defined as 1 unit (1 U). In this technique, the activity of cellulase is a value measured by the cellulase activity measurement method described in the Examples below.

(2) Hemicellulase

Hemicellulase that can be used in the present technique is an enzyme that hydrolyzes hemicellulose, which is one of the cell wall polysaccharides. Hemicellulose is a heteropolysaccharide that is composed of multiple constituent sugars and generally has a branched structure in which the main chain from which the name is derived is supplemented by other constituent sugars that form side chains. Specific examples of hemicellulose include mannan, $\beta$-1,3-1,4-glucan, glucomannan, xylan, xyloglucan, glucuronoxylan, and the like. Hemicellulase is an enzyme that degrades these hemicelluloses.

Specific examples of hemicellulases include xylanase, galactanase, mannanase, galactomannanase, arabinase, $\beta$-glucanase, etc., and one type of these may be used alone, or two or more types may be used in combination. Hemicellulases that can be used in the present technique may be enzymes that further have other functions, so long as they have hemicellulase activity. In the present technique, the powdery feel of the produced plant-base beverage or food can be reduced by allowing hemicellulase to act on a plant-base raw material.

The origin of the hemicellulase that can be used in the present technique is not particularly limited, and may be derived from a microorganism such as a basidiomycete (genera Corticium, Pycnoporus), a filamentous fungus (genera Aspergillus, Humicola, Penicillium, Trichoderma), an actinomycete (genus Streptomyces), or a bacterium (genus Bacillus), or may be an artificially synthesized enzyme. One type of these may be used alone, or two or more types may be used in combination. Among these, in the present technique, hemicellulase derived from a filamentous fungus is preferred, hemicellulase derived from a microorganism of the genus Aspergillus is more preferred, and hemicellulase derived from Aspergillus niger is even more preferred.

The hemicellulase used in the present technique can be prepared from the culture solution of the microorganism from which the above-mentioned hemicellulase is derived. Specific preparation methods include a method of recovering hemicellulase from the culture solution or cells of the above-mentioned microorganism. For example, when a hemicellulase-secreting microorganism is used, the cells can be recovered from the culture solution in advance by filtration, centrifugation, or the like as necessary, and then the enzyme can be separated and/or purified. When a hemicellulase-non-secreting microorganism is used, the cells can be recovered from the culture solution in advance as necessary, and then the cells can be crushed by pressure treatment, ultrasonic treatment, or the like to expose the enzyme, and then the enzyme can be separated and/or purified. As a method for separating and/or purifying the enzyme, a known protein separation and/or purification method can be used without any particular limitation, and examples of the method include centrifugation, UF concentration, salting out, various chromatography methods using ion exchange resins, etc. The separated and/or purified enzyme can be powdered by a drying method such as freeze-drying or vacuum drying, and can also be powdered using an appropriate excipient and/or drying aid in the drying method. In addition, the separated and/or purified enzyme can be liquefied by adding appropriate additives and sterilizing by filtration.

In the present technique, commercially available hemicellulase can also be used, and preferred examples of a commercially available hemicellulase include hemicellulase derived from Aspergillus niger manufactured by Amano Enzyme Inc.

The content of hemicellulase in the powdery feel reducing enzyme agent according to the present technique can be freely set as long as it does not impair the effect of the present technique. The content of hemicellulase can be set to, for example, 1 U or more per 1 g of the plant-base raw material used in the plant-base beverage or food or plant-base beverage or food material to be produced, and from the viewpoint of further enhancing the powdery feel reducing effect, it can be set to preferably 10 U or more, more preferably 50 U or more, even more preferably 100 U or more, and even more preferably 200 U or more.

The upper limit of the hemicellulase content is not particularly limited as long as it does not impair the effects of the present technique, but can be set to, for example, 100,000 U or less, 50,000 U or less, 10,000 U or less, 5,000 U or less, 2,000 U or less, or 1,000 U or less per 1 g of plant-base raw material used in the plant-base beverage or food or plant-base beverage or food material to be produced.

In the present technique, the hemicellulase activity is a value defined by the following method.

[Definition of hemicellulase activity]

The amount of enzyme that generates reducing sugars equivalent to 1 mg of xylose per minute using xylan as a substrate is defined as 100 units (100 U).

(3) Pectinase

Pectinase that can be used in the present technique is an enzyme that hydrolyzes pectin, which is one of the cell wall polysaccharides. Pectin is a complex polysaccharide whose main component is polygalacturonic acid in which galacturonic acid is linked by $\alpha$-1,4-bonds. Specific examples of pectinases include polygalacturonase, pectin lyase, pectin methylesterase, etc., and preferably polygalacturonase. Pectinases that can be used in the present technique may also be enzymes that have other functions as long as they have pectinase activity.

In this technique, the powdery feel of the produced plant-base beverage or food can be reduced by allowing pectinase to act on a plant-base raw material.

The origin of the pectinase that can be used in the present technique is not particularly limited, and may be derived from a microorganism such as a basidiomycete (genus Corticium), a filamentous fungus (genera Aspergillus, Rhizopus, and Trichoderma), a yeast (genera Geotrichum, Trichosporon), an actinomycete (genus Streptomyces), or a bacterium (genus Bacillus), or may be an artificially synthesized enzyme. One type of these may be used alone, or two or more types may be used in combination. Among these, in the present technique, a pectinase derived from a filamentous fungus is preferred, and a pectinase derived from the genus Aspergillus is more preferred.

The pectinase used in the present technique can be prepared from a culture solution of the microorganism from which the above-mentioned pectinase is derived. Specific preparation methods include a method of recovering pectinase from the culture solution or cells of the above-mentioned microorganism. For example, when a pectinase-secreting microorganism is used, the cells can be recovered from the culture solution in advance by filtration, centrifugation, etc., as necessary, and then the enzyme can be separated and/or purified. In addition, when a pectinase-non-secreting microorganism is used, the cells can be recovered from the culture solution in advance as necessary, and then the cells can be crushed by pressure treatment, ultrasonic treatment, etc., to expose the enzyme, and then the enzyme can be separated and/or purified. As a method for separating and/or purifying the enzyme, a known protein separation and/or purification method can be used without any particular limitation, and examples of the method include centrifugation, UF concentration, salting out, various chromatography methods using ion exchange resins, etc. The separated and/or purified enzyme can be powdered by a drying method such as freeze-drying or vacuum drying, and can also be powdered using an appropriate excipient and/or drying aid in the drying method. In addition, the separated and/or purified enzyme can be liquefied by adding appropriate additives and sterilizing by filtration.

In the present technique, commercially available pectinases can also be used, and preferred examples of commercially available products include pectinases derived from the genus Aspergillus manufactured by Amano Enzyme Inc.

The content of pectinase in the powdery feel reducing enzyme agent according to the present technique can be freely set as long as it does not impair the effect of the present technique. The content of pectinase can be set to, for example, 0.01 U or more per 1 g of the plant-base raw material used in the plant-base beverage or food or plant-base beverage or food material to be produced, and from the viewpoint of further enhancing the powdery feel reducing effect, it can be set to preferably 0.1 U or more, more preferably 0.5 U or more, even more preferably 1 U or more, and even more preferably 2 U or more.

The upper limit of the pectinase content is not particularly limited as long as it does not impair the effects of the present technique, but can be set to, for example, 2,000 U or less, 500 U or less, 100 U or less, 50 U or less, 40 U or less, or 30 U or less per 1 g of plant-base raw material used in the plant-base beverage or food or plant-base beverage or food material to be produced.

In the present technique, the pectinase activity is a value defined by the following method.

[Definition of pectinase activity]

Using low methoxyl (LM) pectin as a substrate, the amount of enzyme that reduces the viscosity by 50% in 1 minute is defined as 1 unit (1 U).

(4) Glucoamylase

The enzyme agent for reducing powdery feel according to the present technique may contain glucoamylase. The glucoamylase that can be used in the present technique is an enzyme that has the activity of hydrolyzing the $\alpha$-1,4-glucoside bond of carbohydrates such as starch from the non-reducing end in glucose units. The glucoamylase that can be used in the present technique may be an enzyme that further has other actions as long as it has glucoamylase activity.

In this technique, the sweetness of the produced plant-base beverage or food or plant-base beverage or food material can be enhanced by allowing glucoamylase to act on a plant-base raw material.

The origin of the glucoamylase that can be used in the present technique is not particularly limited, but examples thereof include glucoamylases derived from the genera Aspergillus and Rhizopus. One type of these glucoamylases may be used alone, or several types may be used in combination. Among these glucoamylases, glucoamylases derived from the genus Rhizopus are preferred, and glucoamylases derived from Rhizopus oryzae are more preferred.

Here, "glucoamylase derived from Rhizopus oryzae" means glucoamylase generated by a microorganism (whether a wild type or a mutant type) classified as Rhizopus oryzae, or glucoamylase obtained by a genetic engineering approach using a glucoamylase gene. Therefore, a recombinant generated by a host microorganism into which a glucoamylase gene obtained from Rhizopus oryzae (or a gene modified from said gene) has been introduced also falls under "glucoamylase derived from Rhizopus oryzae".

The glucoamylase that can be used in the present technique can be prepared from the culture solution of the microorganism from which the above-mentioned glucoamylase is derived. Specifically, the glucoamylase can be easily prepared by culturing a glucoamylase-generating bacterium and isolating the glucoamylase using a known means, or by using a gene recombination technology, and the like.

In the present technique, commercially available glucoamylase can also be used, and a preferred example of a commercially available glucoamylase is glucoamylase derived from Rhizopus oryzae manufactured by Amano Enzyme Inc.

The content of glucoamylase in the powdery feel reducing enzyme agent according to the present technique can be freely set as long as it does not impair the effect of the present technique. The content of glucoamylase can be set to, for example, 0.01 U or more per 1 g of the plant-base raw material used in the plant-base beverage or food or plant-base beverage or food material to be produced, preferably 0.1 U or more, more preferably 1 U or more, even more preferably 3 U or more, and even more preferably 5 U or more.

The upper limit of the glucoamylase content is not particularly limited as long as it does not impair the effects of the present technique, but can be set to, for example, 5,000 U or less, 1,000 U or less, 500 U or less, 200 U or less, 100 U or less, or 50 U or less per 1 g of plant-base raw material used in the plant-base beverage or food or plant-base beverage or food material to be produced.

In the present technique, the glucoamylase activity is a value defined by the following method.

[Definition of glucoamylase activity]

Using potato starch as a substrate, the amount of enzyme that brings about an increase in reducing power equivalent to 1 mg of glucose per minute is defined as 1 unit (1 U).

(5) α-amylase

The enzyme agent for reducing powdery feel according to the present technique can also use α-amylase. The α-amylase that can be used in the present technique is an enzyme that acts on starch and mainly hydrolyzes α-1,4 glycosidic bonds. The α-amylase that can be used in the present technique may also be an enzyme that has other functions as long as it has α-amylase activity.

In this technique, by allowing α-amylase to act on a plant-base raw material, starch in the plant-base raw materials used in a plant-base beverage or food or plant-base beverage or food material can be liquefied to improve its solubility.

The origin of α-amylase that can be used in the present technique is not particularly limited, and examples thereof include α-amylases derived from organisms of the genus Aspergillus (e.g., Aspergillus oryzae, Aspergillus niger, etc.) and the genus Bacillus (e.g., Bacillus amyloliquefaciens, Bacillus subtilis, Bacillus Licheniformis, etc.), preferably α-amylases derived from the genus Bacillus or α-amylases derived from Aspergillus, and more preferably α-amylases derived from Bacillus amyloliquefaciens.

The α-amylase that can be used in the present technique can be prepared from the culture solution of the microorganism from which the above-mentioned α-amylase is derived. Specifically, the α-amylase can be easily prepared by culturing an α-amylase-generating bacterium and isolating the α-amylase by a known means, or by using a gene recombination technology, and the like.

In the present technique, commercially available α-amylase can also be used, and a preferred example of a commercially available α-amylase is α-amylase (derived from Bacillus amyloliquefaciens) manufactured by Amano Enzyme Inc.

The content of α-amylase in the powdery feel reducing enzyme agent according to the present technique can be freely set as long as it does not impair the effects of the present technique. The content of α-amylase can be set to, for example, 0.01 U or more per 1 g of the plant-base raw material used in the plant-base beverage or food or plant-base beverage or food material to be produced, and from the viewpoint of further enhancing the effect of improving starch solubility, the content can be set to preferably 0.1 U or more, more preferably 1 U or more, even more preferably 3 U or more, and even more preferably 5 U or more.

The upper limit of the α-amylase content is not particularly limited as long as it does not impair the effects of the present technique, but can be set, for example, to 10,000 U or less, 2,000 U or less, 500 U or less, preferably 300 U or less, more preferably 100 U or less, and even more preferably 50 U or less per 1 g of plant-base raw material used in the plant-base beverage or food or plant-base beverage or food material to be produced.

In the present technique, the α-amylase activity is a value defined by the following method.

[Definition of α-amylase activity]

The amylase activity is measured by iodine reaction using potato starch as a substrate. In other words, the amylase activity is measured by carrying out an enzyme reaction using potato starch as a substrate in a conventional manner, and the amount of enzyme that reduces the color produced by the iodine reaction by 10% in 1 minute is defined as 1 unit (1U).

(6) Other enzymes

The enzyme agent for reducing the powdery feel according to the present technique may further contain β-amylase and/or α-glucosidase.

In this technique, the powdery feel of the produced plant-base beverage or food or plant-base beverage or food material can be further reduced by allowing β-amylase and/or α-glucosidase to act on a plant-base raw material.

The origin of β-amylase that can be used in the present technique is not particularly limited, but examples thereof include β-amylase derived from plants (wheat, soybean) and β-amylase derived from the genus Bacillus, preferably β-amylase derived from the genus Bacillus, and more preferably β-amylase derived from Bacillus Flexus.

The origin of the α-glucosidase that can be used in the present technique is not particularly limited, but preferably, α-glucosidase derived from the genus Aspergillus is used, and more preferably, α-glucosidase derived from Aspergillus niger is used.

The β-amylase and α-glucosidase that can be used in the present technique can be prepared from the culture solution of the microorganisms from which they are derived. Specifically, these can be easily prepared by culturing each generating bacterium and isolating the enzyme using known means, or by using a gene recombination technology, and the like.

In the present technique, commercially available β-amylase and α-glucosidase can also be used. Preferred examples of commercially available products include β-amylase derived from the genus Bacillus Flexus, manufactured by Amano Enzyme Inc., and α-glucosidase derived from Aspergillus niger, manufactured by Amano Enzyme Inc.

The content of β-amylase in the powdery feel reducing enzyme agent according to the present technique can be freely set as long as it does not impair the effect of the present technique. The content of β-amylase can be set to, for example, 0.01 U or more per 1 g of the plant-base raw material used in the plant-base beverage or food or plant-base beverage or food material to be produced, and from the viewpoint of further enhancing the powdery feel reducing effect, it can be set to preferably 0.05 U or more, more preferably 0.1 U or more, even more preferably 0.5 U or more, and even more preferably 1 U or more.

The upper limit of the β-amylase content is not particularly limited as long as it does not impair the effects of the present technique, but can be set to, for example, 1,000 U or less, 200 U or less, 100 U or less, 50 U or less, 30 U or less, or 20 U or less per 1 g of plant-base raw material used in the plant-base beverage or food or plant-base beverage or food material to be produced.

In the present technique, the β-amylase activity is a value defined by the following method.

[Definition of β-amylase activity]

Using potato starch as a substrate, the amount of enzyme that brings about an increase in reducing power equivalent to 1 mg of glucose per minute is defined as 1 unit (1 U).

The content of α-glucosidase in the powdery feel reducing enzyme agent according to the present technique can be freely set as long as it does not impair the effect of the present technique. The content of α-glucosidase can be set to, for example, 1 U or more per 1 g of the plant-base raw material used in the plant-base beverage or food or plant-base beverage or food material to be produced, and from the viewpoint of further enhancing the powdery feel reducing effect, it can be set to preferably 10 U or more, more preferably 100 U or more, even more preferably 300 U or more, and even more preferably 500 U or more, or 1,000 U or more.

The upper limit of the α-glucosidase content is not particularly limited as long as it does not impair the effects of the present technique, but can be set to, for example, 300,000 U or less, 100,000 U or less, 20,000 U or less, 10,000 U or less, 6,000 U or less, or 3,000 U or less per 1 g of plant-base raw material used in the plant-base beverage or food or plant-base beverage or food material to be produced.

In the present technique, the α-glucosidase activity is a value defined by the following method.

[Definition of α-glucosidase activity]

The activity generating 1 mg of glucose from soluble starch at 40°C in 60 minutes is defined as 1 unit (1 U).

(7) Other

The enzyme agent for reducing powdery feel according to the present technique may contain the cell wall polysaccharide degrading enzyme described above, and may further contain one or more enzymes selected from the glucoamylase, α-amylase, β-amylase, and α-glucosidase described above. As long as the effect of the present technique is not impaired, one or more types of other components may be freely selected and contained. Examples of other components that can be used include excipients, pH adjusters, colorants, flavoring agents, disintegrants, lubricants, stabilizers, and other components that are normally used in formulations. Furthermore, components having known or future functions can be used in combination as appropriate depending on the purpose.

2. Plant-base beverage or food, or plant-base beverage or food material

The plant-base beverage or food or plant-base beverage or food material according to the present technique is a plant-base beverage or food or plant-base beverage or food material produced using the enzyme agent for reducing the powdery feel described above. Specific examples of plant-base drinks include oat drinks and rice drinks, more specifically oat milk (also called "oats milk") and rice milk. Specific examples of plant-base foods include plant-base yogurt.

The plant-base beverage or food or plant-base beverage or food material according to the present technique can be exemplified by those that have reduced powdery feel and enhanced sweetness and deep flavor. Treatment with a cell wall polysaccharide degrading enzyme can reduce the powdery feel and improve the solubility of the plant-base raw materials, thereby enhancing the depth of flavor. Furthermore, treatment with α-amylase or glucoamylase can enhance the sweetness.

3. Method for producing plant-base beverage or food or plant-base beverage or food material, and method for reducing powdery feel of plant-base beverage or food or plant-base beverage or food material

The method for producing a plant-base beverage or food or a plant-base beverage or food material according to the present technique includes a step of allowing a cell wall polysaccharide degrading enzyme to act on a plant-base raw material (hereinafter also referred to as a "cell wall polysaccharide degrading enzyme action step"). The cell wall polysaccharide degrading enzyme action step can also be carried out in the plant-base raw material liquefaction step. In addition, depending on the type of plant-base beverage or food or plant-base beverage or food material or the like, it is also possible to carry out general food production steps before, after, or simultaneously with each step, as long as the effect of the present technique is not impaired. In the above-mentioned method for producing a plant-base beverage or food or a plant-base beverage or food material, and the method for reducing the powdery feel of a plant-base beverage or food or a plant-base beverage or food material, a starch liquefaction step ($\alpha$-amylase action step), a glucoamylase action step, a $\beta$-amylase action step, an $\alpha$-glucosidase action step, and a recovery step can also be carried out.

(1) Plant-base raw material

The plant-base raw materials that can be used in the present technique are not particularly limited in origin, type, etc., as long as they do not impair the effects of the present technique, and can be freely selected according to the intended plant-base beverage or food. For example, the examples include beans such as soy beans, green peas, lentils, chickpeas, black beans, broad beans, mung beans, lupine beans, kidney beans, etc.; cereals such as wheat, barley, oats, rice, rye, buckwheat, barnyard millet, millet, teff, etc.; nuts such as almonds, coconuts, peanuts, cashew nuts, hazelnuts, pecan nuts, macadamia nuts, pistachios, walnuts, Brazil nuts, pili nuts, chestnuts, sesame seeds, pine nuts, etc.; hemp seeds (industrial hemp), chia seeds, quinoa, amaranth, canary seeds, flaxseed, etc. In the present technique, one of these may be used alone, or two types or more may be used in combination. Among these raw materials, preferred are cereals, more preferred are oats and rice.

In the present technique, the properties of the plant-base raw materials when subjected to various enzyme treatments are not particularly limited as long as they do not impair the effects of the present technique, but preferably include powder (suspension), liquid, slurry, and paste. Powdery feel is more easily felt when powdery raw materials are used. In the present technique, the cell wall polysaccharide degrading enzyme action step can reduce the powdery feel of a plant-base beverage or food or plant-base beverage or food material, so that a suitable raw material of the present invention is a liquid in which a powdery raw material is suspended.

The plant-base raw material that can be used in the present technique contains carbohydrates and cell wall polysaccharides. The content of carbohydrates contained in a plant-base raw material (based on the weight of the plant-base raw material in a dry state) is not particularly limited, but may be, for example, 20% by weight or more. Preferably, it is 30% by weight or more, more preferably 40% by weight or more, and even more preferably 50% by weight or more. The upper limit of the content range is not particularly limited, but may be, for example, 90% by weight or less, 80% by weight or less, or 70% by weight or less.

The content of cell wall polysaccharides contained in the plant-base raw material that can be used in the present technique is not particularly limited, and is, for example, 0.1% by weight or more. Preferably, it is 0.3 % by weight or more, 0.5% by weight or more, 3% by weight or more, 5% by weight or more, 7% by weight or more, or 9% by weight or more. The upper limit of the content is also not particularly limited, and examples thereof include 90% by weight or less, 70% by weight or less, 50% by weight or less, 30% by weight or less, 20% by weight or less, 15% by weight or less, 10% by weight or less, 5% by weight or less, or 3% by weight or less. To give an example of a specific content, the general content in rice (polished rice) is about 0.3 to 1%, and the general content in oats (oatmeal) is about 5 to 15%.

(2) Cell wall polysaccharide degrading enzyme action step

The cell wall polysaccharide degrading enzyme action step is a step in which a cell wall polysaccharide degrading enzyme is allowed to act on a plant-base raw material. The amount of cell wall polysaccharide degrading enzyme added in the cell wall polysaccharide degrading enzyme action step can be freely set so as not to impair the effect of the present technique. The specific amount of cell wall polysaccharide degrading enzyme added in this technique is the same as the content of the cell wall polysaccharide degrading enzyme in the enzyme agent for reducing powdery feel described above, so a description thereof will be omitted here.

Various conditions for the cell wall polysaccharide degrading enzyme action step can be freely set as long as they do not impair the effects of the present technique. For example, the pH, temperature, action time, etc. can be set according to the physicochemical properties of the cell wall polysaccharide degrading enzyme used, such as the optimum pH, stable pH range, optimum temperature, and temperature stability. The pH can be set, for example, to pH 5.0 to 8.0, preferably pH 5.5 to 7.5, and more preferably pH 5.5 to 7.0. The temperature can be set, for example, to 30°C to 80°C, preferably 40°C to 75°C, and more preferably 50°C to 70°C. The action time can be set, for example, to 1 minute to 12 hours, preferably 3 minutes to 3 hours, and more preferably 5 minutes to 1 hour. The optimal reaction conditions can be determined through preliminary experiments.

(3) α-amylase action step

The α-amylase action step is a step in which α-amylase is allowed to act on a plant-base raw material. The α-amylase action step is mainly performed for the purpose of liquefying starch, but may also be performed for the purpose of improving taste. The amount of α-amylase added in the α-amylase action step can be freely set as long as it does not impair the effect of the present technique. The specific amount of α-amylase added in the present technique is the same as the content of α-amylase in the enzyme agent for reducing the powdery feel described above, and therefore will not be described here.

Various conditions for the α-amylase action step can be freely set as long as they do not impair the effects of the present technique. For example, the pH, temperature, action time, etc. can be set according to the physicochemical properties of the α-amylase used, such as the optimum pH, stable pH range, optimum temperature, and temperature stability. The pH can be set, for example, to pH 5.0 to 8.0, preferably pH 5.5 to 7.5, and more preferably pH 5.5 to 7.0. The temperature can be set, for example, to 30°C to 80°C, preferably 40°C to 75°C, and more preferably 50°C to 70°C. The action time can be set, for example, to 1 minute to 12 hours, preferably 3 minutes to 3 hours, and more preferably 5 minutes to 1 hour. The optimal reaction conditions can be determined through preliminary experiments.

(4) Glucoamylase action step

The glucoamylase action step is a step of allowing glucoamylase to act on a plant-base raw material. The amount of glucoamylase added in the glucoamylase action step can be freely set as long as it does not impair the effect of the present technique. The specific amount of glucoamylase added in the present technique is the same as the content of glucoamylase in the enzyme agent for reducing the powdery feel described above, so the explanation is omitted here.

Various conditions for the glucoamylase action step can be freely set as long as they do not impair the effects of the present technique. For example, the pH, temperature, action time, etc. can be set according to the physicochemical properties of the glucoamylase used, such as the optimum pH, stable pH range, optimum temperature, and temperature stability. The pH can be set, for example, to pH 5.0 to 8.0, preferably pH 5.5 to 7.5, and more preferably pH 5.5 to 7.0. The temperature can be set, for example, to 30°C to 80°C, preferably 40°C to 75°C, and more preferably 50°C to 70°C. The action time can be set, for example, to 1 minute to 12 hours, preferably 3 minutes to 3 hours, and more preferably 5 minutes to 1 hour. The optimal reaction conditions can be determined through preliminary experiments.

(5) β-amylase action step

The β-amylase action step is a step in which β-amylase is allowed to act on a plant-base raw material. The amount of β-amylase added in the β-amylase action step can be freely set as long as it does not impair the effect of the present technique. The specific amount of β-amylase added in the present technique is the same as the content of β-amylase in the above-mentioned powdery feel reducing enzyme agent, so a description thereof will be omitted here.

Various conditions for the β-amylase action step can be freely set as long as they do not impair the effects of the present technique. For example, the pH, temperature, action time, etc. can be set according to the physicochemical properties of the β-amylase used, such as the optimum pH, stable pH range, optimum temperature, and temperature stability. The pH can be set, for example, to pH 5.0 to 8.0, preferably pH 5.5 to 7.5, and more preferably pH 5.5 to 7.0. The temperature can be set, for example, to 30°C to 80°C, preferably 40°C to 75°C, and more preferably 50°C to 70°C. The action time can be set, for example, to 1 minute to 12 hours, preferably 3 minutes to 3 hours, and more preferably 5 minutes to 1 hour. The optimal reaction conditions can be determined through preliminary experiments.

(6) α-glucosidase action step

The α-glucosidase action step is a step in which α-glucosidase is allowed to act on a plant-base raw material. The amount of α-glucosidase added in the α-glucosidase action step can be freely set as long as it does not impair the effect of the present technique. The specific amount of α-glucosidase added in the present technique is the same as the content of α-glucosidase in the above-mentioned powdery feel reducing enzyme agent, so a description thereof will be omitted here.

Various conditions for the α-glucosidase action step can be freely set as long as they do not impair the effects of the present technique. For example, the pH, temperature, action time, etc. can be set according to the physicochemical properties of the α-glucosidase used, such as the optimum pH, stable pH range, optimum temperature, and temperature stability. The pH can be set, for example, to pH 5.0 to 8.0, preferably pH 5.5 to 7.5, and more preferably pH 5.5 to 7.0. The temperature can be set, for example, to 30°C to 80°C, preferably 40°C to 75°C, and more preferably 50°C to 70°C. The action time can be set, for example, to 1 minute to 12 hours, preferably 3 minutes to 3 hours, and more preferably 5 minutes to 1 hour. The optimal reaction conditions can be determined through preliminary experiments.

(7) Recovery step

The recovery step is a step of recovering a plant-base beverage or food or plant-base beverage or food material that has undergone the cell wall polysaccharide degrading enzyme action step. In addition, when the above-mentioned liquefaction step ($\alpha$-amylase action step), glucoamylase action step, $\beta$-amylase action step, $\alpha$-glucosidase action step, etc. are performed, the recovery step recovers the plant beverage or food or plant-base beverage or food material that has undergone the cell wall polysaccharide degrading enzyme action step, and one or more steps selected from the liquefaction step ($\alpha$-amylase action step), glucoamylase action step, $\beta$-amylase action step, and $\alpha$-glucosidase action step. As for the specific recovery method, one or more of the general recovery methods in the production of plant-base beverage or food or plant-base beverage or food materials can be freely combined and used depending on the type of plant-base beverage or food or plant-base beverage or food material to be produced.

The plant-base beverage or food or plant-base beverage or food material that has been subjected to the cell wall polysaccharide degrading enzyme action step is characterized by having reduced powdery feel and/or a reduced amount of insoluble components. Also, the plant-base beverage or food or plant-base beverage or food material that has been subjected to an $\alpha$-amylase action step and/or a glucoamylase action step is characterized by having an enhanced sweetness. Furthermore, the plant-base beverage or food or plant-base beverage or food material that has been subjected to a $\beta$-amylase action step and/or an $\alpha$-glucosidase action step is characterized by having a further reduced powdery feel and/or a further reduced amount of insoluble components.

To summarize the above, the recovery step in the present technique can recover plant-base beverage or food or plant-base beverage or food material with reduced powdery feel and/or plant-base beverage or foods or plant-base beverage or food material with reduced insoluble components, and can also recover plant-base beverages or food or plant-base beverage or food material with enhanced sweetness.

One aspect of the method for producing a plant-base beverage or food or a plant-base beverage or food material and the method for reducing powdery feel of producing a plant-base beverage or food or a plant-base beverage or food material of the present technique includes the following steps (1) and (2). Note that an enzyme inactivation step may be added after step (2).

(1) A step of preparing a plant-base raw material containing cell wall polysaccharides.
(2) A step of treating the prepared raw material with cell wall polysaccharide degrading enzymes.

One aspect of the method for producing a plant-base beverage or food or a plant-base beverage or food material and the method for reducing powdery feel of a plant-base beverage or food or a plant-base beverage or food material of the present technique includes the following steps (1) to (3). Note that an enzyme inactivation step may be added after step (2).

(1) A step of preparing a plant-base raw material containing cell wall polysaccharides.
(2) A step of adding the prepared raw material to cell wall polysaccharide degrading enzymes and treating it with $\alpha$-amylase and glucoamylase.
(3) A step of recovering the plant-base beverage or food or plant-base beverage or food material with reduced powdery feel.

One aspect of the method for producing a plant-base beverage or food or a plant-base beverage or food material and the method for reducing powdery feel of a plant-base beverage or food or a plant-base beverage or food material of the present technique includes the following steps (1) to (3). Note that an enzyme inactivation step may be added after step (2).

(1) A step of preparing a plant-base material containing cell wall polysaccharides.
(2) A step of adding the prepared raw material to cell wall polysaccharide degrading enzymes and treating it with one or more enzymes selected from $\alpha$-amylase, glucoamylase, $\beta$-amylase, and $\alpha$-glucosidase.
(3) A step of recovering the plant-base beverage or food or plant-base beverage or food material with reduced powdery feel.

The treatments with the enzymes used in step (2) may be performed simultaneously or separately. When the treatments with the enzyme are performed separately, the order is not particularly limited. Simultaneous treatment is preferable. When the treatments with the enzymes are performed separately, an enzyme deactivation step may be added between each enzyme treatment as necessary.

EXAMPLES

The present invention will be described in more detail below with reference to examples. Note that the examples described below are representative examples of the present invention, and the scope of the present invention should not be construed as being narrowed by these examples.

1. Plant-base raw material

The plant-base raw materials used in the examples are shown in Table 1 below.

[Table 1]

| Raw material | Origin | Shape | Product name | Manufacturer |
|---|---|---|---|---|
| Oat 1 | Oat | Powder | Oats powder | OFCO Co., Ltd. |
| Oat 2 | Oat | Powder | Oatmeal powder | TOMIZAWA SHOUTEN Inc. |
| Rice 1 | Rice | Powder | Rice powder | Kyoritsu-foods Inc. |
| Rice 2 | Rice | Powder | Alphanized rice powder | Kichijiya Kokuten Co. |
| Rice 3 | Rice | Powder | Alphanized non-glutinous rice powder | OFCO Co., Ltd. |

2. Enzyme used

The various enzymes used in the examples are shown in Table 2 below.

[Table 2]

| Enzyme | Origin | Manufacturer |
|---|---|---|
| Cellulase | Trichoderma viride | Amano Enzyme Inc. |
| Hemicellulase | Aspergillus niger | Amano Enzyme Inc. |
| Pectinase | Aspergillus pulvelentus | Amano Enzyme Inc. |
| $\alpha$-amylase 1 | Bacillus amyloliquefaciens | Amano Enzyme Inc. |
| Glucoamylase 1 | Rhizopus oryzae | Amano Enzyme Inc. |
| Glucoamylase 2 | Aspergillus niger | Amano Enzyme Inc. |
| $\alpha$-amylase 2 | Aspergillus oryzae | Amano Enzyme Inc. |
| $\beta$-amylase | Bacillus flexus | Amano Enzyme Inc. |
| $\alpha$-glucosidase | Aspergillus niger | Amano Enzyme Inc. |

3. Enzyme activity measurement method

[Method for measuring cellulase activity]

The cellulase activity was measured by a method based on the cellulase activity test method in the 9th edition of the Japan's Specifications and Standards for Food Additives.

In advance, about 1 g of carmellose sodium (also known as sodium carboxymethylcellulose) (etherification degree 0.62-0.68) was precisely weighed and dried at 105°C for 4 hours to measure the weight loss. Carmellose sodium corresponding to 0.625 g of the dried product was precisely weighed and placed in a 100 mL Erlenmeyer flask, 50 mL of water was added and heated to dissolve, and after cooling, 10 mL of 1 mol/L acetic acid/sodium acetate buffer (pH 4.5) and water were added to make exactly 100 mL to prepare a substrate solution. 4 mL of the substrate solution was precisely weighed and placed in a 50 mL Nessler tube, and left at $37 \pm 0.5$°C for 10 minutes or more, after which 1 mL of the sample solution was precisely weighed and added, and immediately shaken. This solution was left at $37 \pm 0.5$°C for exactly 30 minutes, and 2 mL of alkaline copper test solution (Somogyi test solution (I)) was precisely added and shaken, and the Nessler tube was plugged, and heated in a water bath for exactly 30 minutes. After cooling with water, 2 mL of Nelson's solution was accurately added and the mixture was shaken well, and further, 3 mL of 0.5 mol/L sodium hydroxide test solution was accurately added and the precipitate was dissolved by shaking, and after leaving the mixture for 20 minutes, 13 mL of 1 mol/L acetic acid/sodium acetate buffer (pH 4.5) was accurately added. 1 mL of this solution was accurately measured, 9 mL of 1 mol/L acetic acid/sodium acetate buffer (pH 4.5) was accurately added, and the mixture was shaken well. The absorbance (A1) of this solution at a wavelength of 750 nm was measured using water as a control. Separately, 1 mL of the sample solution was accurately measured and placed in a 50 mL Nessler tube, 2 mL of alkaline copper test solution (Somogyi test solution (I)) was accurately added and the mixture was shaken, and then 4 mL of substrate solution was accurately measured and added and the mixture was shaken, and the absorbance (A2) thereof was measured in the same manner. Under these conditions, regarding the cellulase activity, the amount of enzyme that brings about an increase in reducing power equivalent to 1 μmole of glucose per minute was defined as 1 unit, and cellulase activity per 1 g or 1 mL of sample (U/g, U/mL) was calculated using the following formula.

[Mathematical Formula 1]

$$\text{Cellulase activity (U/g, U/mL)} = G \times (1/30) \times (1/0.180) \times n$$

G: Amount of glucose generated (mg) calculated from the glucose calibration curve using the (A1-A2/a) value
1/30: Conversion factor per minute
1/0.180: 1 μmole of glucose = 0.180 mg
a: Slope of glucose calibration curve
n: Dilution factor per 1 g or 1 mL of sample

[Method for measuring hemicellulase activity]

The hemicellulase activity was measured by a method based on the hemicellulase activity test method in the 9th edition of the Japan's Specifications and Standards for Food Additives.
An appropriate amount of enzyme was measured, dissolved or uniformly dispersed by adding water, and appropriately diluted to obtain a sample solution. 0.50 g of xylan was measured, about 30 mL of water was added, and the mixture was heated while stirring, and boiled for 3 minutes after it started to boil. After cooling, water was added to this solution to make 50 mL, which was used as a substrate solution. 1 mL of the substrate solution was measured in a test tube, 3 mL of 0.1 mol/L acetate buffer (pH 4.5) was added, and the mixture was heated at 40°C for 10 minutes, after which 1 mL of the sample solution was added, shaken, and heated at 40°C for 30 minutes. 2 mL of Somogyi test solution (III) was added to this solution, mixed, the test tube was plugged, and the mixture was heated in a boiling water bath for 20 minutes, and immediately cooled. After cooling, 1 mL of Nelson test solution was added to this solution, and the mixture was shaken well until the red precipitate was completely dissolved, and the mixture was left at room temperature for about 20 minutes, and then water was added to make 25 mL. This solution was centrifuged at 3,000 rpm at 25°C for 10 minutes, and the supernatant was used as the subject solution. Separately, 1 mL of the substrate solution was measured in a test tube, 3 mL of 0.1 mol/L acetate buffer (pH 4.5) and 2 mL of Somogyi test solution (III) were added, and the mixture was shaken, and then, 1 mL of the sample solution was added, the test tube was plugged, and the mixture was heated in a boiling water bath for 20 minutes and immediately cooled. The same procedure was followed as in the preparation of the subject solution to prepare a comparison solution. The absorbance of the subject solution and the comparison solution was measured at a wavelength of 500 nm using water as a control. The enzyme activity was calculated by taking the amount of enzyme that generates reducing sugar equivalent to 1 mg of xylose per minute under these conditions as 100 units.

[Method for measuring pectinase activity]

The measurement was performed by the following method based on the official compendium. Specifically, 0.700 g of LM pectin was weighed out, placed in about 70 mL of water previously heated to 70-90°C, dissolved, and then cooled. After cooling, the pH was adjusted to 3.50 with 0.1 mol/L citric acid test solution or 0.2 mol/L disodium hydrogen phosphate test solution, and 10 mL of McIlvain's buffer (pH 3.5) and water were added to make 100 mL. A specified amount of sample was weighed out, dissolved by adding sample dilution solution to obtain a specified dilution ratio. In the case of powder, the sample was weighed out into a mortar, a small amount (4-10 mL) of sample dilution solution was added, and the mixture was stirred with a pestle to dissolve.

<Measuring method using a viscometer>

6 mL of LM pectin solution (pH 3.5) and 6 mL of McIlvain's buffer (pH 3.5) were gently placed into tube 1 of the viscometer, which was then placed vertically in a thermostatic water bath at 40±0.5°C and left for 10 to 15 minutes, after which 2 mL of the sample solution was added to initiate the reaction. Immediately after addition, tube 3 was closed with a finger, and air was blown into tube 2 by mouth to mix the contents.

Tube 3 was closed with a finger, and weak suction was applied from tube 2 to prevent air bubbles from entering tube 2, and the liquid level was sucked up to the center of sphere C, and then, suction was stopped, the opening of tube 3 was opened, and the time ti seconds required for the liquid level to flow down from the upper marked line to the lower marked line of sphere B was immediately measured. This operation was repeated five times. Separately, the same operation was performed using 6 mL of LM pectin solution (pH 3.5), 6 mL of McIlvain buffer (pH 3.5), and 2 mL of water to measure the flow time $t_0$ seconds. The same operation was also performed using 14 mL of water to measure the flow time tw seconds.

Under these conditions, the amount of enzyme that reduces the viscosity by 50% in 1 minute was defined as 1 unit, and was calculated using the following formula.

[Mathematical Formula 2]

$$\text{Viscosity reduction rate } (V_{50}) = t_0 - ti/t_0 - tw \times 100$$

Endo PGase force (u/g, u/mL) = $60/V_{50} \times n$
$t_0$: Flow time (seconds) of 6 mL of substrate + 6 mL of buffer + 2 mL of water
ti: Flow time (seconds) of 14 mL of reaction solution
tw: Flow time (seconds) of 14 mL of water
60: Unit conversion factor (1 minute = 60 seconds)
n: Dilution factor per 1 g or 1 mL of sample
$V_{50}$: Time to reach 50% viscosity reduction rate [Ti+ti/2 seconds]

Ti is the elapsed time from the start of the reaction to the start of the i-th viscosity measurement.

A curve was drawn with the viscosity reduction rate on the vertical axis and time [Ti+ti/2 seconds] on the horizontal axis, and the time at which the viscosity reduction rate reached 50% was read.

[Method for measuring α-amylase activity]

10 mL of 1% potato starch substrate solution (0.1 mol/L acetic acid (pH 5.0)) was heated at 37°C for 10 minutes, after which 1 mL of sample solution containing α-amylase was added and immediately shaken. This solution was left to stand at 37°C for 10 minutes, after which 1 mL of this solution was added to 10 mL of 0.1 mol/L hydrochloric acid test solution and immediately shaken. Next, 0.5 mL of this solution was measured, and 10 mL of 0.0002 mol/L iodine test solution (Japanese Pharmacopoeia) was added and shaken, after which the absorbance (AT) at a wavelength of 660 nm was measured using water as a control. Separately, 1 mL of water was added instead of the sample solution, and the procedure was repeated to measure the absorbance (AB). The 0.0002 mol/L iodine test solution (Japanese Pharmacopoeia) was prepared by adding 10 mL of water to 12.7 g of iodine and 25 g of potassium iodide, mixing thoroughly, adding water to make 100 mL, and then diluting 2500-fold with water. The α-amylase activity was calculated using the following formula. The amount of enzyme that reduces the color of potato starch caused by iodine by 10% in 1 minute was defined as 1 unit (1 U).

[Mathematical Formula 3]

$$\text{α-Amylase activity (U/g, U/mL)} = \{(AB-AT)/AB\} \times 1/W$$

AT: Absorbance of reaction solution
AB: Absorbance of blank solution
W: Amount of sample in 1 mL of sample solution (g or mL)

[Method for measuring glucoamylase activity]

The glucoamylase titer (activity value) was measured by the following method.

<Glucoamylase (GA) activity>

The measurement was performed according to the following method in accordance with Method 4 of the Glucoamylase Activity Test in the 9th Edition of the Japan's Specifications and Standards for Food Additives.

0.50 g of the enzyme sample was weighed out and diluted with water to an appropriate concentration to prepare a sample solution. Potato starch was dried at 105°C for 2 hours in advance, and 1.0 g of the dried product was weighed out, 20 mL of water was added, and 5 mL of sodium hydroxide test solution (2 mol/L) was gradually added while stirring to form a glue. The glue-like starch was heated in a water bath with stirring for 3 minutes, and then 25 mL of water was added, and after cooling, hydrochloric acid test solution (2 mol/L) and hydrochloric acid test solution (0.1 mol/L) were added to neutralize, and 10 mL of 1 mol/L acetic acid/sodium acetate buffer (pH 4.5) was added, and water was added to make 100 mL to prepare a substrate solution.

10 mL of the substrate solution was measured, heated at 37°C for 10 minutes, 1 mL of the sample solution was added and immediately shaken, heated at 37°C for 10 minutes, 4 mL of Fehling's test solution was added and gently shaken, heated in a water bath for 15 minutes, cooled to 25°C or lower, and 2 mL of potassium iodide test solution and 2 mL of sulfuric acid (1 volume part of sulfuric acid diluted with water to 6 volume parts) were added to prepare a subject solution. Separately, a comparison solution was prepared by the same procedure as in the preparation of the subject solution, but using 10 mL of water instead of the substrate solution. The liberated iodine in the subject solution and the comparison solution was titrated with 0.05 mol/L sodium thiosulfate solution. One to two drops of soluble starch test solution was added near the end of the titration, and the end point of the titration was determined as the time when the blue color disappeared. Under these conditions, the amount of enzyme that causes an increase in reducing power equivalent to 1 mg of glucose per minute was defined as 1 unit (1U), and glucoamylase activity was calculated from the following formula.

[Mathematical Formula 4]

$$\text{Glucoamylase activity (U/g)} = \text{amount of glucose (mg)} \times 1/10 \times 1/M$$

$$\text{Amount of glucose (mg)} = (b - a) \times 1.6 \times f$$

a: Titration value of the subject solution (mL)
b: Titration value of the comparison solution (mL)
1.6: 1 mL of 0.05 mol/L sodium thiosulfate solution is equivalent to 1.6 mg of glucose.
1/10: Unit conversion factor for reaction time (min)
M: Amount of enzyme sample in 1 mL of sample solution (g or mL)
f: Factor of 0.05 mol/L sodium thiosulfate solution

[Method for measuring β-amylase activity]

The measurement was performed according to the method described in the Japan's Specifications and Standards for Food Additives (9th edition). Specifically, potato starch was used as a substrate, and it was dried at 105°C for 2 hours in advance, and 1.0 g of the dried material was weighed out, 20 mL of water was added, and 5 mL of sodium hydroxide test solution (2 mol/L) was gradually added while stirring to form a glue. Next, the mixture was heated in a water bath while stirring for 3 minutes, and then 25 mL of water was added. After cooling, the mixture was neutralized by adding hydrochloric acid test solution (2 mol/L) and hydrochloric acid test solution (0.1 mol/L), 10 mL of 1 mol/L acetic acid/sodium acetate buffer (pH 5.0) was added, and water was further added to make 100 mL to prepare a substrate solution.

10 mL of the substrate solution was measured, heated at 37°C for 10 minutes, 1 mL of sample solution was added, and the mixture was immediately shaken, and after heating at the same temperature for 10 or 30 minutes, 4 mL of Fehling's test solution was added, and the mixture was gently shaken, heated in a water bath for 15 minutes, cooled to 25°C or lower, and 2 mL of 30% potassium iodide solution and 2 mL of sulfuric acid (1→6) were added to prepare a subject solution. Fehling's test solution was prepared just before use by mixing 1 volume of copper solution and 1 volume of alkaline tartrate solution, in which the copper solution was made by measuring 34.66 g of fine crystals of copper (II) sulfate pentahydrate and dissolving it in water to make 500 mL, and the alkaline tartrate solution was made by measuring 173 g of (+)-potassium sodium tartrate tetrahydrate and 50 g of sodium hydroxide and dissolving it in water to make 500 mL. Separately, the same procedure was followed as in the preparation of the subject solution using 10 mL of water instead of the substrate solution to prepare a comparison solution. The liberated iodine in the subject solution and the comparison solution was titrated with 0.05 mol/L sodium thiosulfate solution. One to two drops of soluble starch test solution was added near the end of the titration, and the end point of the titration was determined as the time when the blue color disappeared.

The amount of enzyme that brings about an increase in reducing power equivalent to 1 mg of glucose per minute is defined as 1 unit (1 U), and was calculated using the following formula.

$\beta$-amylase activity (U/g, U/mL) = amount of glucose (mg) $\times$ 1/10 $\times$ 1/M amount of glucose (mg) = (b-a) $\times$ 1.6 $\times$ f
[Mathematical Formula 5]

a: Titration value of enzyme reaction solution (mL), b: Titration value of blank solution (mL)
1.6: 1 mL of 0.05 mol/L sodium thiosulfate solution is equivalent to 1.6 mg of glucose.
1/10: Unit conversion factor for reaction time (min)
M: Amount of sample in 1 mL of sample solution (g or mL)
f: Factor of 0.05 mol/L sodium thiosulfate solution (for quantitative analysis)

[Method for measuring $\alpha$-glucosidase activity]

The $\alpha$-glucosidase activity was measured by Method 2 of the $\alpha$-glucosidase Activity Test in the 9th Edition of the Japan's Specifications and Standards for Food Additives, and 1 unit was defined as the amount of enzyme that generates 1 $\mu$g of glucose in 2.5 mL of reaction solution in 60 minutes under these conditions.

[Mathematical Formula 6]

$$\alpha\text{-glucosidase activity (U/g)} = (E60-E0) \times G \times 2.5/0.1 \times n/0.5$$

E60: Absorbance of subject solution
E0: absorbance of the comparison solution
G: Amount of glucose ($\mu$g) when the absorbance difference is 1.000 (calculated from the glucose calibration curve)
2.5: Volume of reaction system liquid (mL)
0.1: Amount of reaction solution collected (mL)
0.5: Amount of sample solution collected (mL)
n: sample dilution factor

4. Experimental Examples

<Experimental Example 1>

In Experimental Example 1, the effect of cell wall polysaccharide degrading enzymes in reducing powdery feel was examined.

(1) Experimental method

To 3.6 g of the above-mentioned plant-base raw material, an enzyme agent was added in the amount shown in Tables 3 and 4 below, 30 mL of hot water at 60°C was added, and the mixture was allowed to stand at 60°C for 30 minutes to obtain plant-base milk. Note that the above-mentioned plant-base raw material powder was heated in hot water at 60°C for 30 minutes without enzyme treatment to obtain plant-base milk of the reference example.

(2) Measurement and Evaluation

[Measurement of Brix value]

After heating at 60°C for 30 minutes, the Brix value was measured using a Brix meter.

[Solubility Measurement]

To evaluate the liquefaction of the powder by the enzyme of the plant-base milk, 10 mL of the plant-base milk was taken after heating at 60°C for 30 minutes and centrifuged at 10,000 rpm for 15 minutes. The solubility of the plant-base protein material by the enzyme was calculated based on the weight of the precipitate after centrifugation.

[Sensory evaluation]

Three panelists evaluated the changes in taste caused by the enzymes in the plant milk. "Powdery feel," "sweetness," and "flavor of material" were rated on a three-point scale according to the following criteria. In addition, for the examples using oat 2 and rice 1 to 3 as raw materials, "depth (complexity)" was also rated on a three-point scale according to the following criteria. Note that "flavor of material" refers to the flavor unique to oats in the case of oats, and the flavor unique to rice in the case of rice.

(a) Powdery feel

3 points: Reduced compared to the reference example
2 points: Slightly reduced compared to the reference example
1 point: No change compared to the reference example

(b) Sweetness

3 points: Sweeter than the reference example
2 points: Slightly sweeter than the reference example
1 point: No change compared to the reference example

(c) Flavor of material

3 points: The flavor of the material is stronger than in the reference example
2 points: The flavor of the material is slightly stronger than in the reference sample.
1 point: No change compared to the reference example

(d) Depth

3 points: Deeper flavor than the reference sample (more complex flavor)
2 points: Slightly deeper than the reference example
1 point: No change compared to the reference example

(3) Results

The results are shown in Tables 3 and 4 below.

[Table 3]

| | | Reference Example | Comparative Example | Example | | | | | | | |
|---|---|---|---|---|---|---|---|---|---|---|---|
| | | 1 | 1 | 1 | 2 | 3 | 4 | 5 | 6 | 7 | 8 |
| Used enzyme U/g-raw material | α-amylase 1 | - | 13 | 13 | 13 | 13 | 13 | 13 | 13 | 13 | 13 |
| | Glucoamylase 1 | - | 16 | 16 | 16 | 16 | 16 | 16 | 16 | 16 | 16 |
| | Cellulas e | - | - | 1.4 | 2.8 | 5.6 | - | - | 1.4 | 1.4 | 1.4 |
| | Hemicellulase | - | - | - | - | - | 900 | - | 450 | - | 450 |
| | Pectinase | - | - | - | - | - | - | 12 | - | 6 | 6 |
| Brix (%) | Oat 1 | 1.4 | 8.0 | 8.3 | 8.4 | 8.8 | 8.6 | 8.5 | - | - | - |
| | Oat 2 | 0.6 | 8.1 | 8.7 | - | - | - | - | 8.9 | 8.7 | 9.2 |
| Precipitate (g) | Oat 1 | 4.0 | 1.7 | 1.6 | 1.5 | 1.5 | 1.5 | 1.5 | - | - | - |
| | Oat 2 | 5.6 | 2.1 | 2.0 | - | - | - | - | 2.0 | 2.0 | 1.9 |
| Powdery feel | Oat 1 | 1.0 | 2.5 | 3.0 | 3.0 | 3.0 | 3.0 | 3.0 | - | - | - |
| | Oat 2 | 1.0 | 2.5 | 3.0 | - | - | - | - | 3.0 | 3.0 | 3.0 |

(continued)

| | | Reference Example | Comparative Example | Example | | | | | | | |
|---|---|---|---|---|---|---|---|---|---|---|---|
| | | 1 | 1 | 1 | 2 | 3 | 4 | 5 | 6 | 7 | 8 |
| Sweetness | Oat 1 | 1.0 | 1.5 | 1.8 | 2.0 | 2.5 | 2.5 | 2.5 | - | - | - |
| | Oat 2 | 1.0 | 1.8 | 2.0 | - | - | - | - | 3.0 | 2.5 | 3.0 |
| Flavor of material | Oat 1 | 1.0 | 1.0 | 2.0 | 2.0 | 1.8 | 2.0 | 2.0 | - | - | - |
| | Oat 2 | 1.0 | 1.5 | 2.5 | - | - | - | - | 3.0 | 2.0 | 3.0 |
| Depth | Oat 2 | 1.0 | 1.5 | 2.5 | - | - | - | - | 3.0 | 2.5 | 2.5 |

[Table 4]

| | | Reference Example | Comparative Example | Example | | | | | | | | | |
|---|---|---|---|---|---|---|---|---|---|---|---|---|---|
| | | 2 | 2 | 9 | 10 | 11 | 12 | 13 | 14 | 15 | 16 | 17 | 18 |
| Used enzyme U/g-raw material | α-amylase 1 | - | 13 | 13 | 13 | 13 | 13 | 13 | 13 | 13 | 13 | 13 | 13 |
| | Glucoamylase 1 | - | 16 | 16 | 16 | 16 | 16 | 16 | 16 | - | 16 | 16 | 16 |
| | Glucoamylase 2 | - | - | - | - | - | - | - | - | 32 | - | - | - |
| | Cellulase | - | - | 2.8 | - | - | 2.8 | 1.4 | 5.6 | 2.8 | 2.8 | 2.8 | 2.8 |
| | Hemicellulase | - | - | - | 900 | - | 900 | - | - | - | - | - | - |
| | Pectinase | - | - | - | - | 12 | 12 | - | - | - | - | - | - |
| | β-amylase | - | - | - | - | - | - | - | - | - | 6.5 | - | - |
| | α-glucosidase | - | - | - | - | - | - | - | - | - | - | 600 | - |
| | α-amylase 2 | - | - | - | - | - | - | - | - | - | - | - | 70 |
| Brix (%) | Rice 1 | 2.9 | 9.7 | 9.4 | 9 | 9.1 | 9.5 | 9.6 | 9.1 | 9.7 | 9.5 | 9.4 | 9.6 |
| | Rice 2 | 9.1 | 10.4 | 10.1 | 10 | 10 | 10.2 | 10.2 | 10.2 | 10.2 | 10 | 10.1 | 10.2 |
| | Rice 3 | 9.1 | 10.4 | 10.1 | 10 | 10 | 10.2 | 10.2 | 10.2 | 10.2 | 10 | 10.1 | 10.2 |
| Precipitate (9) | Rice 1 | 4.9 | 2.0 | 2.0 | 1.9 | 1.8 | 2.2 | 2.0 | 1.7 | 1.9 | 2.0 | 1.6 | 1.8 |
| | Rice 2 | 4.7 | 1.1 | 1.0 | 0.9 | 0.9 | 0.9 | 0.8 | 1.1 | 1.0 | 1.4 | 1.0 | 1.1 |
| | Rice 3 | 2.8 | 2.3 | 2.3 | 2.2 | 2.1 | 2.2 | 2.3 | 2.2 | 2.1 | 2.1 | 2.3 | 2.2 |
| Powdery feel | Rice 1 | 1.0 | 1.0 | 3.0 | 3.0 | 3.0 | 3.0 | 2.0 | 3.0 | 3.0 | 3.0 | 3.0 | 3.0 |
| | Rice 2 | 1.0 | 1.0 | 2.0 | 2.0 | 2.0 | 3.0 | 2.0 | 3.0 | 3.0 | 3.0 | 3.0 | 3.0 |
| | Rice 3 | 1.0 | 1.0 | 2.0 | 2.0 | 2.0 | 3.0 | 1.5 | 3.0 | 3.0 | 3.0 | 3.0 | 3.0 |
| Sweetness | Rice 1 | 1.0 | 2.0 | 3.0 | 2.0 | 3.0 | 3.0 | 3.0 | 2.0 | 3.0 | 2.5 | 3.0 | 3.0 |
| | Rice 2 | 1.0 | 2.0 | 2.0 | 2.0 | 2.0 | 3.0 | 3.0 | 3.0 | 3.0 | 3.0 | 3.0 | 3.0 |
| | Rice 3 | 1.0 | 2.0 | 2.5 | 2.0 | 2.0 | 2.5 | 2.0 | 2.0 | 2.0 | 1.5 | 2.0 | 2.0 |
| Flavor of material | Rice 1 | 1.0 | 2.0 | 2.5 | 2.0 | 3.0 | 2.0 | 2.0 | 2.5 | 2.0 | 1.0 | 2.0 | 3.0 |
| | Rice 2 | 1.0 | 1.0 | 2.0 | 2.0 | 2.0 | 2.0 | 1.0 | 2.0 | 2.0 | 2.0 | 3.0 | 3.0 |
| | Rice 3 | 1.0 | 1.5 | 2.0 | 2.0 | 1.5 | 2.0 | 1.5 | 2.0 | 2.0 | 2.0 | 2.0 | 1.5 |

(continued)

| | | Reference Example | Comparative Example | Example | | | | | | | | | |
|---|---|---|---|---|---|---|---|---|---|---|---|---|---|
| | | 2 | 2 | 9 | 10 | 11 | 12 | 13 | 14 | 15 | 16 | 17 | 18 |
| Depth | Rice 1 | 1.0 | 1.0 | 2.0 | 2.0 | 2.0 | 2.0 | 1.0 | 3.0 | 2.5 | 1.5 | 2.0 | 3.0 |
| | Rice 2 | 1.0 | 1.5 | 2.0 | 2.0 | 2.0 | 2.0 | 2.0 | 2.0 | 2.0 | 1.5 | 2.0 | 2.0 |
| | Rice 3 | 1.0 | 1.0 | 2.0 | 1.0 | 1.5 | 1.5 | 2.0 | 2.5 | 2.0 | 1.5 | 2.0 | 1.5 |

(4) Observations

As shown in Examples 1 to 15 in Tables 3 and 4, the effects of increasing Brix, reducing precipitates, reducing powdery feel, enhancing sweetness, and changing taste were confirmed for all raw materials by treatment with cell wall polysaccharide degrading enzymes. In addition, as shown in Examples 16 to 18 in Table 4, it was confirmed that the powdery feel was further improved by further treatment with β-amylase, a-glucosidase, and α-amylase 2.

**Claims**

1. An enzyme agent for reducing powdery feel of a plant-base beverage or food or a plant-base beverage or food material, comprising a cell wall polysaccharide degrading enzyme.

2. A plant-base beverage or food or a plant-base beverage or food material, in which the enzyme agent for reducing powdery feel according to claim 1 is used.

3. The plant-base beverage or food according to claim 2, wherein the plant-base beverage or food is oat milk or rice milk.

4. A method for producing a plant-base beverage or food or a plant-base beverage or food material, comprising a step of allowing a cell wall polysaccharide degrading enzyme to act on a plant-base raw material.

5. A method for reducing powdery feel of a plant-base beverage or food or a plant-base beverage or food material, comprising a step of allowing a cell wall polysaccharide degrading enzyme to act on a plant-base raw material.

## INTERNATIONAL SEARCH REPORT

| International application No. |
| --- |
| **PCT/JP2023/033012** |

**A. CLASSIFICATION OF SUBJECT MATTER**

*A23L 5/00*(2016.01)i; *A23C 11/10*(2021.01)i; *A23L 2/00*(2006.01)i; *A23L 2/38*(2021.01)i; *A23L 2/52*(2006.01)i; *A23L 7/10*(2016.01)i; *A23L 7/104*(2016.01)i; *A23L 11/60*(2021.01)i; *A23L 11/65*(2021.01)i; *C12N 9/26*(2006.01)i; *C12N 9/42*(2006.01)i
FI:   A23L5/00 J; A23C11/10; A23L2/00 B; A23L2/38 J; A23L2/52; A23L7/10 H; A23L7/104; C12N9/26 Z; C12N9/42

According to International Patent Classification (IPC) or to both national classification and IPC

**B. FIELDS SEARCHED**

Minimum documentation searched (classification system followed by classification symbols)

A23L5/00; A23C11/10; A23L2/00; A23L2/38; A23L2/52; A23L7/10; A23L7/104; A23L11/60; A23L11/65; C12N9/26; C12N9/42

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
| --- | --- | --- |
| X | WO 2022/071418 A1 (AMANO ENZYME INC.) 07 April 2022 (2022-04-07)<br>    examples 1-8 | 2-4 |
| Y | examples 1-8, paragraph [0036] | 1, 5 |
| Y | JP 2017-079801 A (ASAHI BREWERIES LTD) 18 May 2017 (2017-05-18)<br>    paragraph [0063] | 1, 5 |
| X | JP 2012-521775 A (DANISCO A/S) 20 September 2012 (2012-09-20)<br>    claims 24, 28, 38, paragraphs [0001], [0047]-[0048] | 2-4 |
| Y | claims 24, 28, 38, paragraphs [0001], [0047]-[0048] | 1, 5 |

☐ Further documents are listed in the continuation of Box C.    ☑ See patent family annex.

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| **10 November 2023** | **21 November 2023** |

| Name and mailing address of the ISA/JP | Authorized officer |
| --- | --- |
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (January 2015)

**INTERNATIONAL SEARCH REPORT**
Information on patent family members

International application No.

**PCT/JP2023/033012**

| Patent document cited in search report | | | Publication date (day/month/year) | Patent family member(s) | | | Publication date (day/month/year) |
|---|---|---|---|---|---|---|---|
| WO | 2022/071418 | A1 | 07 April 2022 | CN | 114304276 | A | |
| JP | 2017-079801 | A | 18 May 2017 | (Family: none) | | | |
| JP | 2012-521775 | A | 20 September 2012 | WO | 2010/115754 | A1 | |
| | | | | claims 24, 28, 38, pp. 1, 9 | | | |
| | | | | EP | 2413715 | A1 | |
| | | | | AR | 76037 | A | |
| | | | | CA | 2756932 | A | |
| | | | | AU | 2010233935 | A | |
| | | | | CN | 102421302 | A | |
| | | | | CN | 105248510 | A | |
| | | | | BR | PI1012562 | A | |
| | | | | US | 2012/0034343 | A1 | |

Form PCT/ISA/210 (patent family annex) (January 2015)

23

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2021040553 A
- JP 2018075029 A
- JP 2015084687 A
- JP 2004121135 A